# EUROPEAN PATENT APPLICATION

(11) **EP 4 248 840 A1**
(43) Date of publication of application: **27.09.2023**
(21) Application number: 21894971.7
(22) Date of filing: 04.11.2021
(51) Int. Cl.: A61B 5/00, A61B 5/145, G16H 50/20, G06N 20/00

(54) **PHOTOACOUSTIC DIAGNOSTIC DEVICE AND METHOD, USING LASER COMBINATION HAVING SINGLE WAVELENGTH**

(30) Priority: 17.11.2020 KR 20200154019
(71) Applicant: HME Square Co., Ltd., Daejeon 34138 (KR)
(72) Inventor: KHANG, Yoonho, Yongin-si Gyeonggi-do 16822 (KR)
(74) Representative: dompatent von Kreisler Selting Werner - Partnerschaft von Patent- und Rechtsanwälten mbB
(86) International application number: PCT/KR2021/015898
(87) International publication number: WO 2022/108200

(57) **Abstract**

The present invention relates to a photoacoustic diagnotic apparatus and method using a combination of single-wavelength laser lights, and more particularly, to a photoacoustic diagnostic apparatus and method enabling the miniaturization of photoacoustic equipment because they irradiate a subject with a combination of single-wavelength laser lights. The photoacoustic diagnostic apparatus according to one embodiment of the present invention may comprise: a light source configured to irradiate a subject with light consisting of a combination of single-wavelength laser lights; a detection unit configured to acquire sound emitted by the light; and a control unit configured to calculate the concentration of glucose in the blood of the subject by analyzing the sound.

## Description

### Technical Field

The present invention relates to a photoacoustic diagnosis apparatus and method using a combination of single-wavelength laser lights, and more particularly, to a photoacoustic diagnostic apparatus and method enabling the miniaturization of photoacoustic equipment because they irradiate a subject with a combination of single-wavelength laser lights.

### Background Art

Diabetes is a disease that affects one in 10 of the global adult population and places a significant economic burden on individuals, families, health systems, and countries. If the level of glucose in the blood of a diabetic patient is not maintained, the diabetic patient has serious complications such as cardiovascular disease, kidney disease, and diabetic foot, which cause great inconvenience to the patient's life and can threaten life. Therefore, blood glucose levels need to be regularly monitored, and high blood glucose levels need to be controlled immediately.

In general, a blood glucose level is determined from an invasively obtained blood sample using an electrochemical sensor containing an enzyme. In this case, obtaining blood through a process such as piercing a finger with a needle can cause great inconvenience to a diabetic patient whose blood glucose level needs to be measured several times during the day, and also causes high risk of infection.

In addition, non-invasive blood glucose level measurement methods developed so far include Raman spectroscopy, diffuse reflection spectroscopy, thermal emission spectroscopy, near-infrared absorption spectroscopy, millimeter-wave/terahertz spectroscopy, transdermal impedance spectroscopy, sonophoresis, and iontophoresis techniques. However, these measurement methods have a problem in that accuracy is lowered depending on skin tissue secretions or skin conditions.

As a way to solve this problem, Korean Patent Application Publication No. 10-2019-0063446 discloses a method of predicting blood levels in the body using photoacoustic imaging. However, the method disclosed in the above patent document has problems in that a subject is irradiated with laser light with continuously changing wavelengths to measure blood glucose levels, and for this reason, the size of the laser and optical equipment increases, and in that it is difficult to manufacture a wearable device.

### DISCLOSURE

### Technical Problem

The present invention has been made in order to solve the above-mentioned problems, and an object of the present invention is to provide a photoacoustic diagnostic apparatus and method making it possible to reduce the size of laser and optical equipment because they use an optimal combination of single-wavelength lasers capable of distinguishing blood glucose from other substances.

### Technical Solution

According to one embodiment of the present invention, there is provided a photoacoustic diagnostic apparatus that uses a combination of single-wavelength lasers.

The photoacoustic diagnostic apparatus according to one embodiment of the present invention may comprise: a light source configured to irradiate a subject with light consisting of a combination of single-wavelength laser lights; a detection unit configured to acquire sound emitted by the light; and a control unit configured to calculate the concentration of glucose in the blood of the subject by analyzing the sound.

In the photoacoustic diagnostic apparatus according to one embodiment of the present invention, the light may comprise at least one of a first laser light having at least one wavelength in the range of 1,400 nm to 1,520 nm, a second laser light having at least one wavelength in the range of 1,520 nm to 1,720 nm, and a third laser light having at least one wavelength in the range of 1,720 nm to 1,850 nm.

In the photoacoustic diagnostic apparatus according to one embodiment of the present invention, the control unit may calculate the concentration of glucose in the blood using machine learning.

According to one embodiment of the present invention, there is provided a photoacoustic diagnostic method that uses a combination of single-wavelength lasers.

The photoacoustic diagnostic method according to one embodiment of the present invention may comprise: a light irradiation step of irradiating a subject with light consisting of a combination of a plurality of single-wavelength laser lights through a light source; a sound detection step of acquiring sound, emitted by the light, through a detection unit; and a blood glucose concentration analysis step of calculating the concentration of glucose in the blood of the subject by analyzing the sound.

In the photoacoustic diagnostic method according to one embodiment of the present invention, the light in the light irradiation step may comprise at least one of a first laser light having at least one wavelength in the range of 1,400 nm to 1,520 nm, a second laser light having at least one wavelength in the range of 1,520 nm to 1,720 nm, and a third laser light having at least one wavelength in the range of 1,720 nm to 1,850 nm.

In the photoacoustic diagnostic method according to one embodiment of the present invention, the blood glucose concentration analysis step may further comprise: a training step of training an artificial neural network using data obtained by repeatedly performing the light irradiation step and the sound detection step; and an artificial intelligence analysis step of calculating the concentration of glucose in the blood of the subject using the artificial neural network.

According to one embodiment of the present invention, a computer-readable recording medium having recorded thereon a program for executing the above-described method.

### Advantageous Effects

The photoacoustic diagnostic apparatus and method according to the present invention have advantages in that they use an optimal wavelength combination capable of distinguishing glucose contained in blood from other substances, making it possible to minimize the size of optical equipment used in the photoacoustic diagnostic apparatus, and in that they may be implemented as a wearable device.

The effects obtainable in the present disclosure are not limited to the above-mentioned effects, and other effects not mentioned herein will be clearly understood by those skilled in the art from the flowing description.

### Brief Description of Drawings

FIG. 1 is a block diagram showing a photoacoustic diagnostic apparatus according to one embodiment of the present invention.
FIG. 2 is a graph showing examples of the light absorption coefficients of substances contained in the human body depending on the wavelength of laser light.
FIG. 3 shows an example of a combination of single-wavelength lasers, which is used in a photoacoustic diagnostic apparatus according to one embodiment of the present invention.
FIG. 4 is a flow chart showing a photoacoustic diagnostic method according to one embodiment of the present invention.
FIG. 5 is a flow chart showing a blood glucose concentration analysis step in the photoacoustic diagnostic method according to one embodiment of the present invention.

### Best Mode

According to one embodiment of the present invention, there is provided a photoacoustic diagnostic apparatus that uses a combination of single-wavelength lasers.

The photoacoustic diagnostic apparatus according to one embodiment of the present invention may comprise: a light source configured to irradiate a subject with light consisting of a combination of single-wavelength laser lights; a detection unit configured to acquire sound emitted by the light; and a control unit configured to calculate the concentration of glucose in the blood of the subject by analyzing the sound.

In the photoacoustic diagnostic apparatus according to one embodiment of the present invention, the light may comprise at least one of a first laser light having at least one wavelength in the range of 1,400 nm to 1,520 nm, a second laser light having at least one wavelength in the range of 1,520 nm to 1,720 nm, and a third laser light having at least one wavelength in the range of 1,720 nm to 1,850 nm.

In the photoacoustic diagnostic apparatus according to one embodiment of the present invention, the control unit may calculate the concentration of glucose in the blood using machine learning.

According to one embodiment of the present invention, there is provided a photoacoustic diagnostic method that uses a combination of single-wavelength lasers.

The photoacoustic diagnostic method according to one embodiment of the present invention may comprise: a light irradiation step of irradiating a subject with light consisting of a combination of a plurality of single-wavelength laser lights through a light source; a sound detection step of acquiring sound, emitted by the light, through a detection unit; and a blood glucose concentration analysis step of calculating the concentration of glucose in the blood of the subject by analyzing the sound.

In the photoacoustic diagnostic method according to one embodiment of the present invention, the light in the light irradiation step may comprise at least one of a first laser light having at least one wavelength in the range of 1,400 nm to 1,520 nm, a second laser light having at least one wavelength in the range of 1,520 nm to 1,720 nm, and a third laser light having at least one wavelength in the range of 1,720 nm to 1,850 nm.

In the photoacoustic diagnostic method according to one embodiment of the present invention, the blood glucose concentration analysis step may further comprise: a training step of training an artificial neural network using data obtained by repeatedly performing the light irradiation step and the sound detection step; and an artificial intelligence analysis step of calculating the concentration of glucose in the blood of the subject using the artificial neural network.

According to one embodiment of the present invention, there is provided a computer-readable recording medium having recorded thereon a program for executing the above-described method.

### Mode for Invention

Hereinafter, embodiments of the present invention will be described in detail with reference to the accompanying drawings so that the present invention can be easily carried out by those skilled in the art to which the present invention pertains. However, the present invention may be embodied in various different forms and is not limited to the embodiments described herein. In drawings, parts irrelevant to the description are omitted for the simplicity of explanation, and like parts are denoted by like reference numerals throughout the specification.

The terms used in this specification will be briefly described, and the present invention will be described in detail.

The terms used in the present specification are currently widely used general terms selected in consideration of their functions in the present disclosure, but they may change depending on the intents of those skilled in the art, precedents, or the advents of new technology. Additionally, in certain cases, there may be terms arbitrarily selected by the applicant, and in this case, their meanings are described in a corresponding description part of the present disclosure. Accordingly, terms used in the present disclosure should be defined based on the meaning of the term and the entire contents of the present disclosure, rather than the simple term name.

Throughout the present specification, it is to be understood that when any part is referred to as "comprising" any component, it does not exclude other components, but may further comprise other components, unless otherwise specified. In addition, terms such as ".... unit" and "module" described in the specification mean a unit that processes at least one function or operation, which may be implemented in hardware or software, or a combination of hardware and software. In addition, it is to be understood that, when any part is referred to as being "connected" to another part, it can be connected directly to the other part or intervening elements may be present.

Hereinafter, the present invention will be described in detail with reference to the accompanying drawings.

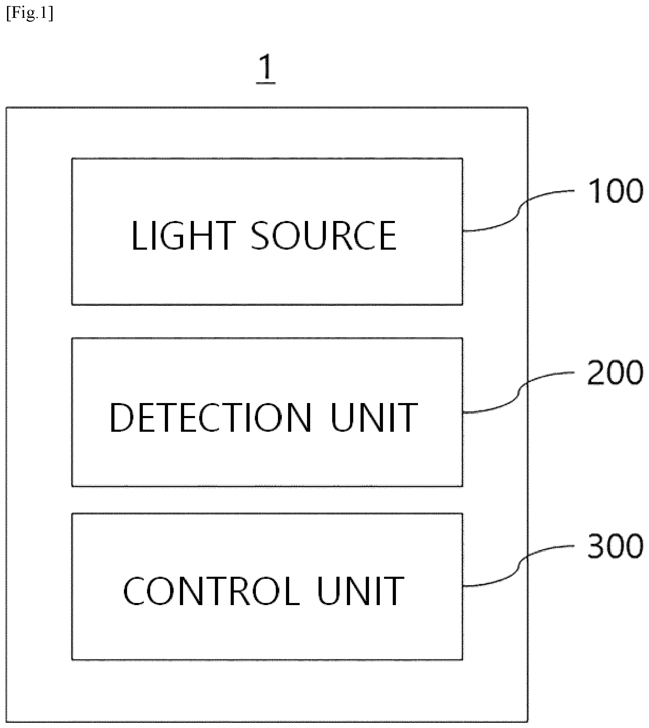

FIG. 1 is a block diagram showing a photoacoustic diagnostic apparatus according to one embodiment of the present invention.

Referring to FIG. 1, a photoacoustic diagnostic apparatus 1 according to one embodiment of the present invention may comprise: a light source 100 configured to irradiate a subject with light consisting of a combination of single-wavelength laser lights; a detection unit 200 configured to acquire sound emitted by the light; and a control unit 300 configured to calculate the concentration of glucose in the blood of the subject by analyzing the sound.

The light source 100 may output a plurality of single-wavelength laser lights, and the laser lights may be infrared rays. That is, in the present invention, the light source 100 does not output light while sequentially increasing or decreasing the wavelength in the near-infrared or mid-infrared band, but outputs a combination of a plurality of laser lights having specific wavelengths.

The light output from the light source 100 irradiates a subject and vibrates molecules contained in the subject, and ultrasonic waves are emitted by the vibration of the molecules.

The detection unit 200 may comprise: a sound receiving unit that receives sound emitted from the subject by the light; and a measuring unit that measures a shape change of the sound receiving unit.

The detection unit 200 may employ a piezo method or a MEMS (Micro Electro Mechanical System) method. In the piezo method, a potential is formed in a piezoelectric material by the pressure of ultrasonic waves, and the voltage, which is the potential difference, is measured to measure the ultrasonic waves. In the MEMS method, the shape of a membrane is changed by the pressure of ultrasonic waves, and the shape change is measured to measure the ultrasonic waves.

The control unit 300 may control the light source 100 or the detection unit 200 to irradiate the subject with light, acquire sound emitted thereby, and calculate the concentration of glucose in the blood.

The control unit 300 may calculate the concentration of glucose in the blood by directly analyzing the sound, or may train an artificial intelligence neural network using result data obtained through repeated experiments, and calculate the concentration of glucose in the blood using the trained artificial neural network.

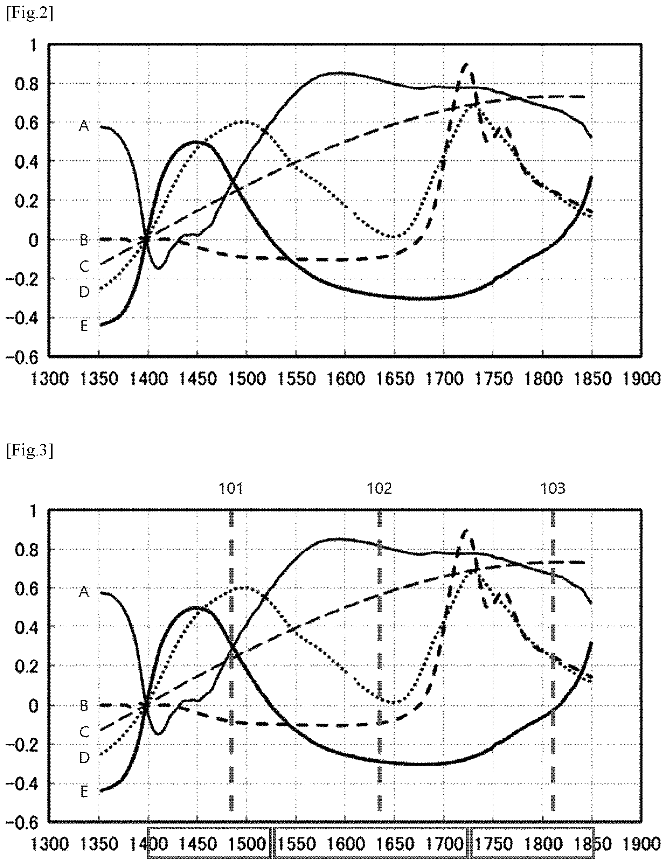

FIG. 2 is a graph showing examples of the light absorption coefficients of substances contained in the human body depending on the wavelength of laser light.

Referring to FIG. 2, it can be seen that, in addition to blood glucose, various substances absorbing laser light are contained in the human body.

In FIG. 2, the x-axis represents wavelength (nm), and the y-axis represents absorption spectra (λ). In addition, in FIG. 2, A, B, C, D and E represent the light absorption spectra of glucose, fat, a baseline, protein, and water, respectively, depending on wavelength.

That is, according to the present invention, using the fact that wavelength-dependent light absorption coefficient is different between substances, it is possible to find an optimal wavelength combination for distinguishing blood glucose from other substances, and calculate the concentration of blood glucose using the same. This makes it possible to greatly reduce the size of the optical equipment.

FIG. 3 shows an example of a combination of single-wavelength lasers, which is used in the photoacoustic diagnotic apparatus according to one embodiment of the present invention.

Referring to FIG. 3, in the photoacoustic diagnostic apparatus according to one embodiment of the present invention, the light may comprise at least one of a first laser light 101 having at least one wavelength in the range of 1,400 nm to 1,520 nm, a second laser light 102 having at least one wavelength in the range of 1,520 nm to 1,720 nm, and a third laser light 103 having at least one wavelength in the range of 1,720 nm to 1,850 nm.

For example, the photoacoustic diagnostic apparatus according to one embodiment of the present invention may comprise the light source 100 that outputs a combination of the first laser light 101 having a wavelength of 1,480 nm, the second laser light 102 having a wavelength of 1,640 nm, and the third laser light 103 having a wavelength of 1,810 nm.

The wavelengths of the first 101 to third laser lights 103 may be selected as optimal wavelengths capable of distinguishing blood glucose from other substances, within the limited ranges.

The criteria used when selecting the optimal wavelengths include the point at which the magnitude difference in the light absorption coefficient between substances is the largest, and the point at which the light absorption coefficient of any one of the substances other than blood glucose is equal to the light absorption coefficient of blood glucose.

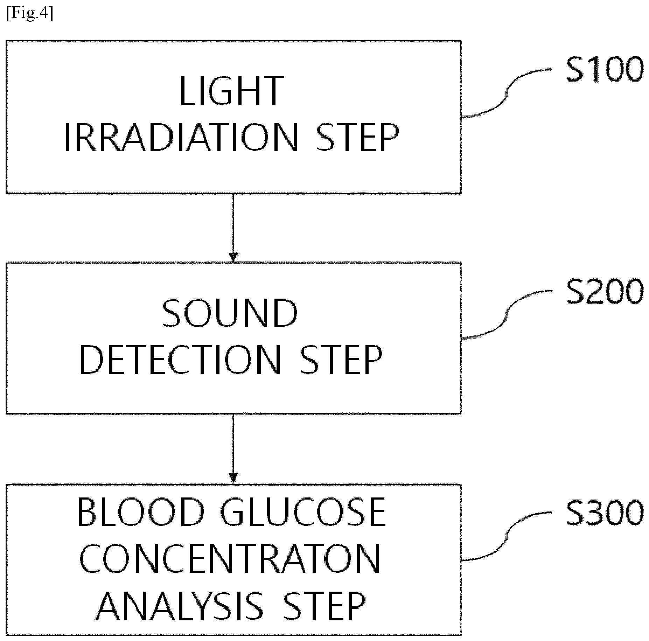

FIG. 4 is a flow chart of a photoacoustic diagnostic method according to one embodiment of the present invention.

Referring to FIG. 4, the photoacoustic diagnostic method according to one embodiment of the present invention may comprise: a light irradiation step S100 of irradiating a subject with light consisting of a combination of a plurality of single-wavelength laser lights through the light source 100; a sound detection step S200 of acquiring sound, emitted by the light, through the detection unit 200; and a blood glucose concentration analysis step S300 of calculating the concentration of glucose in the blood of the subject by analyzing the sound.

In the photoacoustic diagnostic method according to one embodiment of the present invention, the light in the light irradiation step S100 may comprise at least one of the first laser light 101 having at least one wavelength in the range of 1,400 nm to 1,520 nm, the second laser light 102 having at least one wavelength in the range of 1,520 nm to 1,720 nm, and the third laser light 103 having at least one wavelength in the range of 1,720 nm to 1,850 nm.

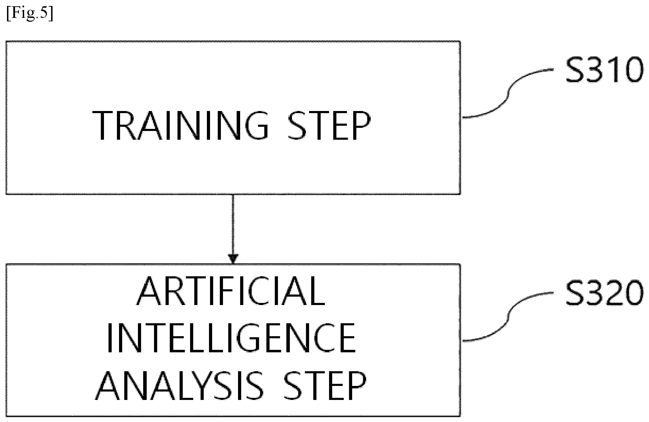

FIG. 5 is a flow chart showing the blood glucose concentration analysis step S300 in the photoacoustic diagnostic method according to one embodiment of the present invention.

Referring to FIG. 5, the blood glucose concentration analysis step S300 in the photoacoustic diagnostic method according to one embodiment of the present invention may further comprise: a training step S310 of training an artificial neural network using data obtained by repeatedly performing the light irradiation step S100 and the sound detection step S200; and an artificial intelligence analysis step S320 of calculating the concentration of glucose in the blood of the subject using the artificial neural network.

Contents regarding the above-described apparatus may be applied to the method according to one embodiment of the present invention. Accordingly, in the method, description of the same contents as those of the above-described apparatus is omitted.

The above description of the present invention is exemplary, and those of ordinary skill in the art will appreciate that the present invention can be easily modified into other specific forms without departing from the technical spirit or essential characteristics of the present invention. Therefore, it should be understood that the exemplary embodiments described above are exemplary in all aspects and are not restrictive. For example, each component described to be of a single type can be implemented in a distributed manner. Likewise, components described to be distributed can be implemented in a combined manner.

The scope of the present invention is defined by the following claims rather than by the above detailed description. It shall be understood that all modifications and embodiments conceived from the meaning and scope of the claims and equivalents thereto are included in the scope of the present invention.

## Claims

1. A photoacoustic diagnostic apparatus comprising:
a light source configured to irradiate a subject with light consisting of a combination of single-wavelength laser lights;
a detection unit configured to acquire sound emitted by the light; and
a control unit configured to calculate the concentration of glucose in the blood of the subject by analyzing the sound.

2. The photoacoustic diagnostic apparatus of claim 1, wherein the light comprises at least one of a first laser light having at least one wavelength in the range of 1,400 nm to 1,520 nm, a second laser light having at least one wavelength in the range of 1,520 nm to 1,720 nm, and a third laser light having at least one wavelength in the range of 1,720 nm to 1,850 nm.

3. The photoacoustic diagnostic apparatus of claim 1, wherein the control unit calculates the concentration of glucose in the blood using machine learning.

4. A photoacoustic diagnostic method comprising:
a light irradiation step of irradiating a subject with light consisting of a combination of a plurality of single-wavelength laser lights through a light source;
a sound detection step of acquiring sound, emitted by the light, through a detection unit; and
a blood glucose concentration analysis step of calculating the concentration of glucose in the blood of the subject by analyzing the sound.

5. The photoacoustic diagnostic method of claim 4, wherein the light in the light irradiation step comprises at least one of a first laser light having at least one wavelength in the range of 1,400 nm to 1,520 nm, a second laser light having at least one wavelength in the range of 1,520 nm to 1,720 nm, and a third laser light having at least one wavelength in the range of 1,720 nm to 1,850 nm.

6. The photoacoustic diagnostic method of claim 4, wherein the blood glucose concentration analysis step comprises: a training step of training an artificial neural network using data obtained by repeatedly performing the light irradiation step and the sound detection step; and an artificial intelligence analysis step of calculating the concentration of glucose in the blood of the subject using the artificial neural network.

7. A computer-readable recording medium having recorded thereon a program for executing the method of any one of claims 4 to 6.
